(19) Europäisches Patentamt European Patent Office Office européen des brevets

(11) **EP 4 779 642 A1**

(12) **EUROPEAN PATENT APPLICATION**

(43) Date of publication:
**22.07.2026 Bulletin 2026/30**

(21) Application number: **25152904.6**

(22) Date of filing: **20.01.2025**

(51) International Patent Classification (IPC):
**G16B 20/00** (2019.01)   **G16B 40/20** (2019.01)
**G16H 50/20** (2018.01)   **G16H 50/30** (2018.01)

(52) Cooperative Patent Classification (CPC):
**G16H 50/30; G01N 33/6806; G01N 33/6893;**
**G16B 20/00; G16B 40/20; G16H 50/20;**
G01N 2800/042

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Cerascreen GmbH**
**19059 Schwerin (DE)**

(72) Inventors:
• **Blum, Wilfried**
**21335 Lüneburg (DE)**
• **Anchang, Charles Gwellem**
**Potsdam (DE)**

(74) Representative: **Simandi, Claus**
**Simandi Patentanwälte**
**Kurfürstendamm 45**
**10719 Berlin (DE)**

(54) **METHOD FOR THE PREDICTION OF DIABETES USING AMINO ACIDS**

(57)   The present invention relates to a method of determining whether a subject is at risk of developing diabetes using a classification system and biomarkers, in particular amino acids for predicting and stratifying diabetes.

Figure 9. Predictors of diabetes

**Description**

The present invention relates to a method of determining whether a subject is at risk of developing diabetes using a classification system and amino acids as biomarkers for predicting and stratifying diabetes.

Diabetes mellitus is a complex metabolic disorder characterized by chronic hyperglycemia and significant disturbances in carbohydrate, fat, and amino acid metabolism (World Health Organization. (2016). Global report on diabetes. World Health Organization. https://apps.who.int/iris/handle/10665/204871). The global prevalence of diabetes is rising, making it crucial to develop effective tools for early detection and monitoring of disease progression. Understanding the metabolic changes that differentiate diabetic from non-diabetic individuals is critical for elucidating the pathophysiology of the disease and identifying biomarkers for disease progression and personalized management (Kahn SE, Cooper ME, Del Prato S. "Pathophysiology and treatment of type 2 diabetes: perspectives on the past, present, and future." Lancet, 2014.).

Risk prediction for type 2 diabetes (T2D) remains suboptimal even after the introduction of global risk assessment by various scores. The known risk factors for T2D include age, sex, anthropometric (obesity, blood lipids and blood pressure), metabolic (blood pressure, liver enzymes and uric acid), socioeconomic (leukocyte count, C-reactive protein and adiponectin) and life style (physical inactivity, dietary components, smoking and alcohol) variables. An improvement of risk prediction for T2D is crucial to the identification of low- and high-risk individuals who could benefit from targeted preventive measures.

Consequently, there is growing interest in developing predictive models that integrate these diverse risk factors to improve the early detection of diabetes.

The present invention comprises a computer-implemented method for determining whether a subject is at risk of developing diabetes, as well as for monitoring processes in which a large number of monitoring parameters are recorded in parallel. The method comprises at least a data acquisition step in which at least one measurement data set is received, as well as at least one subsequent data acquisition step in which at least one further measurement data set is received. The first data acquisition step comprises the at least one starting value measurement data set. Each measurement data set can provide a biomarker, i.e. amino acids profile/pattern of a subject and further parameter. The method may further comprise a data evaluation step. An evaluation is the processing of (raw) data from an experiment with the (raw) data from the at least one starting value measurement data set and the at least one further measurement data set to generate concrete knowledge. The data evaluation step comprises, for each measurement data set, determining a starting value of the respective amino acids profile/pattern of a subject and further parameter on the basis of the measurement data set. Furthermore, the data evaluation step comprises quantifying the change of the respective amino acids pattern of a subject and further parameter with respect to the starting value by means of a mathematical distance measure by determining at least one further subsequent data acquisition step with the at least one further measurement data set. The method may further comprise a data output step in which the change in the risk of developing diabetes is graphically displayed for each measurement data set.

Various classification systems such as machine learning approaches for data analysis and data mining have been widely explored for recognizing patterns and enabling the extraction of important information contained within large data bases in the presence of other information. Learning machines comprise algorithms that may be trained to generalize using data with known classifications.

Trained learning machine algorithms may then be applied to predict the outcome in cases of unknown outcomes, i.e., to classify data according to learned patterns. Machine learning methods, which include neural networks, hidden Markov models, belief networks and kernel based classifiers such as support vector machines, are useful for problems characterized by large amounts of data, noisy patterns and the absence of general theories.

Many successful approaches to pattern classification, regression and clustering problems rely on kernels for determining the similarity of a pair of patterns. These kernels are usually defined for patterns that can be represented as a vector of real numbers. For example, the linear kernel, radial basis kernel and polynomial kernel all measure the similarity of a pair of real vectors. Such kernels are appropriate when the data can best be represented in this way, as a sequence of real numbers. The choice of kernel corresponds to the choice of representation of the data in the feature space. In many applications, the patterns have a greater degree of structure. These structures can be exploited to improve the performance of the learning algorithm. Examples of the types of structured data that commonly occur in machine learning applications are strings, documents, trees, graphs, such as websites or chemical molecules, signals, such as microarray expression profiles, spectra, images, spatio-temporal data, relational data and biochemical concentrations, amongst others.

Classification systems have been used in the medical field. For example, methods of diagnosing and predicting the occurrence of a medical condition have been proposed using various computer systems and classification systems such as support vector machines. For a specific review on diabetes, see Dashdondov et al. (Dashdondov, K.; Lee, S.; Erdenebat, M.-U. Enhancing Diabetes Prediction and Prevention through Mahalanobis Distance and Machine Learning Integration. Appl. Sci. 2024, 14, 7480).

The specific use of biomarkers such as amino acids is not disclosed by Dashdondov et al. Amnio acids are known as biomarker for the diagnosis of diabetes (e.g. CN106979982A).

The technical problem underlying the present invention is to identify alternative and/or improved means and methods for identifying subjects at risk of developing diabetes mellitus including its prediction or monitoring or stratification, wherein metabolites responsible for the metabolic progression of diabetes are involved.

This invention highlights the urgent need to improve early detection and intervention strategies for diabetes due to its metabolic progression.

In particular, it describes the integration of different data preparation techniques, machine learning algorithms and the role of outlier detection using Mahalanobis distance (Mahalanobis, P.C. (1936) "On the Generalised Distance in

Statistics.". Sankhya A 80 (Suppl 1), 1-7 (2018). https://doi.org/10.1007/s13171-019-00164-5) and related pseudotime.

The solution to this technical problem is achieved by providing the embodiments characterized in the claims.

The term "diabetes" as used herein refers to "type 1 or type 2 diabetes mellitus"

"Type 2 diabetes mellitus (T2D)" as used herein relates to a condition characterized by the development of the symptoms of increased blood glucose levels believed to be the result of beta-cell dysfunction and insulin resistance. The criteria (as established by the World Health Organization) for the diagnosis of T2D include (a) fasting plasma glucose >=126 mg/dl and/or (b) two-hour post-glucose load plasma >=200 mg/dl during an oral glucose tolerance test with 75 g glucose and (c) HbA1c >=6.5%.

The term "type 1 diabetes mellitus" as used here refers to an autoimmune disease in which a dysfunction of the beta cells is genetically caused.

The invention relates to various methods of detection, identification, prediction, stratification, prognosis and diagnosis of diabetes using preferably amino acids as biomarkers. These methods involve determining the amounts of amino acids and using these amino acids in a classification system to determine the likelihood that an individual has diabetes or non-diabetes.

The term "stratification" or "risk stratification" according to the invention, comprises finding diabetes patients or subjects, particularly those having diabetic sequelae, with the worse prognosis, for the purpose of intensive diagnosis and therapy/treatment (of sequelae) of diabetes mellitus, with the goal of allowing as advantageous a course of the diabetes mellitus as possible.

For this reason, it is particularly advantageous that a reliable prediction, diagnosis and/or (risk) stratification can take place by means of the methods according to the invention. The method according to the invention allows clinical decisions that lead to a more rapid diagnosis, particularly of the diabetic sequelae. Such clinical decisions also comprise further treatment using medications, for the treatment or therapy of diabetes mellitus.

In another preferred embodiment of the method according to the invention, diagnosis and/or risk stratification take place for prognosis, for prophylaxis, for early detection and detection by means of differential diagnosis, for assessment of the degree of severity, and for assessing the course of diabetes mellitus as an accompaniment to therapy.

Metabolites, in particular amino acids were identified by measuring the amounts of amino acids as biomarkers in the sample of patients from populations who had been diagnosed with diabetes as well as patients who had not been diagnosed with diabetes.

The term "determining the amount" as used herein in the context of metabolites refers to any method that can be employed to quantify the presence of one or more amino acid(s). The person skilled in the art is aware of experimental protocols that are suitable to determine the amount of one or more amino acid(s) as metabolite or biomarker. For example, methods such as nuclear magnetic resonance (NMR) and/or mass spectrometry alone or in combination with, e.g., gas or liquid chromatography can be employed. Mass spectrometry and its use for determining the concentration of metabolites in a sample is well known in the art. Mass spectrometry includes, for example, tandem mass spectrometry, matrix assisted laser desorption ionization (MALDI) time-of-flight (TOF) mass spectrometry, MALDI-TOF-TOF mass spectrometry, MALDI Quadrupole-time-of-flight (Q-TOF) mass spectrometry, electrospray ionization (ESI)-TOF mass spectrometry, ESI-Q-TOF, ESI-TOF-TOF, ESI-ion trap mass spectrometry, ESI Triple quadrupole mass spectrometry, ESI Fourier Transform mass spectrometry (FTMS), MALDI-FTMS, MALDI-Ion Trap-TOF, and ESI-Ion Trap TOF. Liquid chromatography mass spectrometry combines the physical separation capabilities of liquid chromatography (LC) or high-performance liquid chromatography (HPLC), with the mass analysis capabilities of mass spectrometry (MS). HPLC provides the advantage over LC that has a shorter analysis time and better resolution of analytes. This consequently increases selectivity, precision and accuracy of MS.

The assessment of the "amount" of said one or more amino acid(s) is the decisive factor in the process of diagnosing a risk to develop T2D in accordance with the method of the invention. However. The amounts of metabolites generally and normally vary from subject to subject depending on age, sex and/or condition, inter alia, to a certain extent. As such, the amounts can be normalized. Several strategies for the normalisation of amino acid(s) concentrations are known in the art, including, without being limiting, normalising against the concentration of an internal reference, which is determined in the same sample, normalisation against sample size, normalisation against total metabolite or amino acids amount or normalisation against an artificially introduced molecule of known amount. In addition, normalisation may also be carried out by adjusting the obtained values by patient-specific features such as e.g. age, BMI, hormone status, nutritional factors (e.g. fasting) or time (circadian rhythm). Such a normalization can be conducted by means of data preprocessing, whereby any measurement data set is involved.

Preferably, the method comprises a data preprocessing step. Furthermore, the data preprocessing step may comprise performing a centering, normalization and/or scaling of the at least one measurement data set.

Data preprocessing is understood here as the mathematical processing of raw data with the aim of preparing the actual evaluation. This can, for example, be a stray light correction, an increase in the signal-to-noise ratio or a simple reformatting of recorded measurement data sets. Include raw data so that the data fed in during a subsequent evaluation can be correctly processed by the algorithm. Suitable data preprocessing can be used in particular to increase the quality of the results of the method according to the invention. The data preprocessing preferably takes place after the data acquisition step of the method according to the invention.

In a preferred embodiment of the invention the preprocessing on the data is conducted with propensity score weighting as outlined in the examples.

A pattern as used here is a discernible regularity or structured arrangement that repeats or organises in a predictable manner, either in physical forms, namely the pattern of metabolites, particularly amino acids, due to the collected 'amounts' of said one or more amino acid(s). An amino acid pattern can have a specific shape in data.

The term "metabolic progression" as used herein refers to the series of biochemical and physiological changes that occur in a person's metabolism over time, either as a result of natural ageing, disease processes or environmental and lifestyle factors, that lead to the onset, exacerbation or management of diabetes, including the pre-diabetic state (insulin resistance), onset of diabetes and advanced diabetes.

The metabolic progression of diabetes involves a complex interplay between insulin resistance, beta-cell dysfunction and the systemic effects of hyperglycaemia. Understanding this progression helps in early detection, intervention and personalised treatment to slow or reverse the course of the disease.

The term "sample" as used herein refers to a biological sample, such as, preferably, fluids, including serum, plasma, whole blood, which has been isolated or obtained from an individual or subject.

For example, blood can be drawn into suitable containers (e.g., tubes such as S-Monovette® serum tubes (SARSTEDT AG & Co., Nümbrecht, Germany)) followed by one or more gentle inversions of the containers, and letting the samples rest for 30 minutes at room temperature to obtain complete coagulation. For serum collection, centrifugation of blood, e.g. at 2750 g and 15° C. for 10 minutes, can be performed. Serum can then be separated and, if desired, filled into containers, e.g., synthetic straws, for storage, such as in liquid nitrogen (-196° C.), until the execution of the analyses.

As used herein, a" "biomarker" or "marker" is a biological molecule that can be objectively measured as a characteristic indicator of the physiological status of a biological system.

As used herein, a "subject" means any mammal, such as, for example, a human or individual. In many embodiments, the subject will be a human patient or individual having, or at-risk of having diabetes. As used herein, the term "plurality of subjects" may be 2, 3, 4, 5, 6, 7, 8, 9, 10, 100, 1000 or more subjects.

In accordance with this invention, samples are collected from subjects in a manner which ensures that the amino acids as biomarkers in the sample is proportional to the concentration of that biomarker in the subject from which the sample is collected. Measurements are made so that the measured value is proportional to the concentration of the biomarker in the sample. Selecting sampling techniques and measurement techniques which meet these requirements is within the ordinary skill of the art.

The invention relates to predicting or stratifying diabetes based on multiple, continuously distributed amino acids as biomarkers. For some classification systems {e.g., support vector machines), prediction may be a three-step process. In the first step, a classifier is built by describing a pre-determined set of data. This is the "learning step" and is carried out on "training" data.

The term "training data," as used herein generally refers to data that can be input into models, statistical models, algorithms and any system or process able to use existing data to make predictions.

The inventors applied this enhanced data set to several machine-learning classifiers, including Extreme Gradient Boosting (XGBoost), Naive Bayes (NB), K-Nearest Neighbors (KNN), Random Forest (RF), and Decision Trees (DTs). In a preferred embodiment of the invention the classifier refers to Random Forest (RF).

In a further preferred embodiment of the invention the classifier refers to Distance-Based Classification Methods. Examples of Distance-Based Classification Methods are not limited to k-Nearest Neighbors (k-NN), Centroid-Based Classifiers, Minimum Distance Classifier and Support Vector Machines (SVMs) with Distance-Based Kernels.

In a preferred embodiment of the invention the mathematical distance measure. preferably Mahalanobis distance can be applied in distance-based classification methods to determine whether a point is an outlier. Mahalanobis distance is a distance metric used to measure the dissimilarity between a point and a distribution, or between two points in a multivariate space, accounting for the correlations among the variables. Effective outlier detection is crucial for improving the data quality and predictive model performance. Mahalanobis distance, a multivariate distance measure, offers a robust method for outlier detection by accounting for correlations among variables and the overall covariance structure of the data.

If the Mahalanobis distance is ordered in increasing order using the robust mean and covariance matrix of the amino acid profiles of non-diabetic or healthy subjects as the baseline, then the Mahalanobis distance effectively represents pseudotime (progression).

The robust mean and covariance matrix are calculated to minimize the influence of outliers, ensuring that the Mahalanobis distance reflects the deviation from a healthy state.

Moreover, other mathematical distance measure can be selected from the following group: Euclidean distance, Manhattan distance, Pearson distance and/or Gower distance. However, Mahalanobis distance (measure) is preferred.

By normalising and ordering these above-mentioned distances, the inventors found a pseudotime metric that surprisingly reflects the metabolic progression from non-diabetic to diabetic subjects, wherein a pseudotime value identifies a subject at risk.

Therefore, the present invention refers to a method or use for predicting, monitoring or stratifying diabetes mellitus comprising

> (a) providing, for each of a plurality of subjects a sample and determining the amount of at least one amino acid in each sample
> (b) providing an amino acid pattern of at least one amino acid from (a) and providing a data set configured to combine the said amino acid pattern with at least one feature of the subject selected from the group consisting of sex, age, height, weight, body mass index
> (c) performing a preprocessing on the data set of (b), preferably using at least propensity score weighting
> (d) calculating one or more mathematical distances, in particular Mahalanobis distances from the data set of (c); and
> (e) i.) identifying a subject at risk or ii.) differentiating between a non-diabetic and a diabetic subject.

In a further preferred embodiment step d.) comprises the following step and calculating one or more pseudotime values therefrom.

In a further embodiment of the invention the risk reflects the metabolic progression of diabetes.

In a further preferred embodiment of the invention the inventive method comprises a further step (f) having a classification system, wherein the classification system is a machine-learning classifier (supra), and wherein the data set is configured from (b) and / or (d).

Therefore, the present invention refers to a method or use for predicting, monitoring or stratifying diabetes mellitus comprising

> (a) providing, for each of a plurality of subjects a sample and determining the amount of at least one amino acid in each sample
> (b) providing an amino acid pattern of at least one amino acid from (a) and providing a data set configured to combine the said amino acid pattern with at least one feature of the subject selected from the group consisting of sex, age, height, weight, body mass index
> (c) performing a preprocessing on the data set of (b), preferably using at least propensity score weighting
> (d) calculating one or more mathematical distances, in particular Mahalanobis distances from the data set of (c); and
> (e) using a classification system, wherein the classification system is a machine-learning classifier, and wherein the data set is configured from (b) and/or (d), and classifying i.) a subject at risk or ii.) differentiating between a non-diabetic and a diabetic subject.

In a preferred embodiment of the invention the amino acid is at least one selected from the group consisting of phenylalanine, tyrosine, tryptophan, histidine, alanine, ornithine, arginine. However, phenylalanine is most preferred.

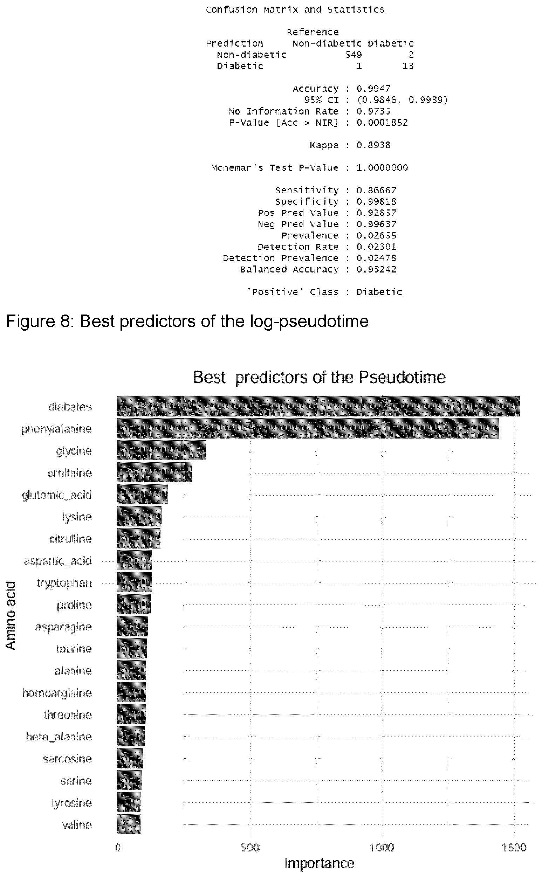

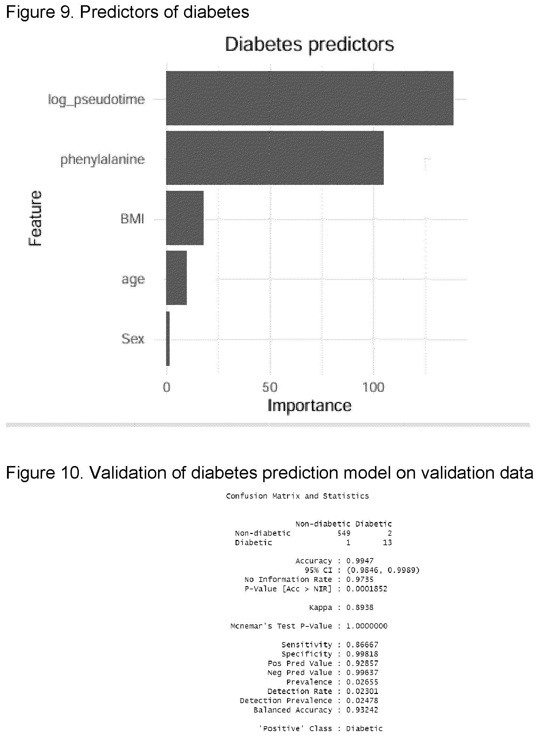

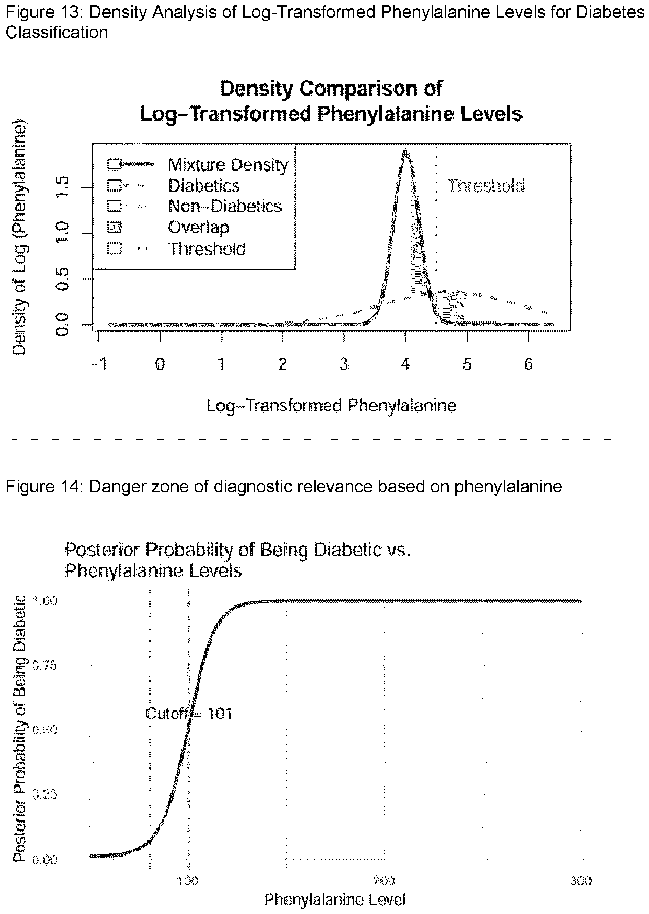

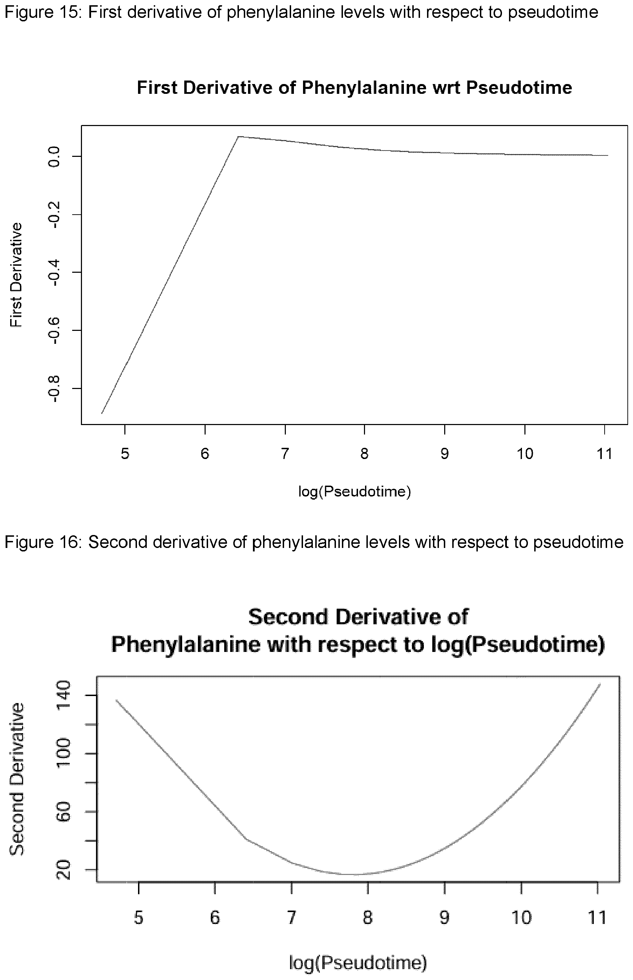

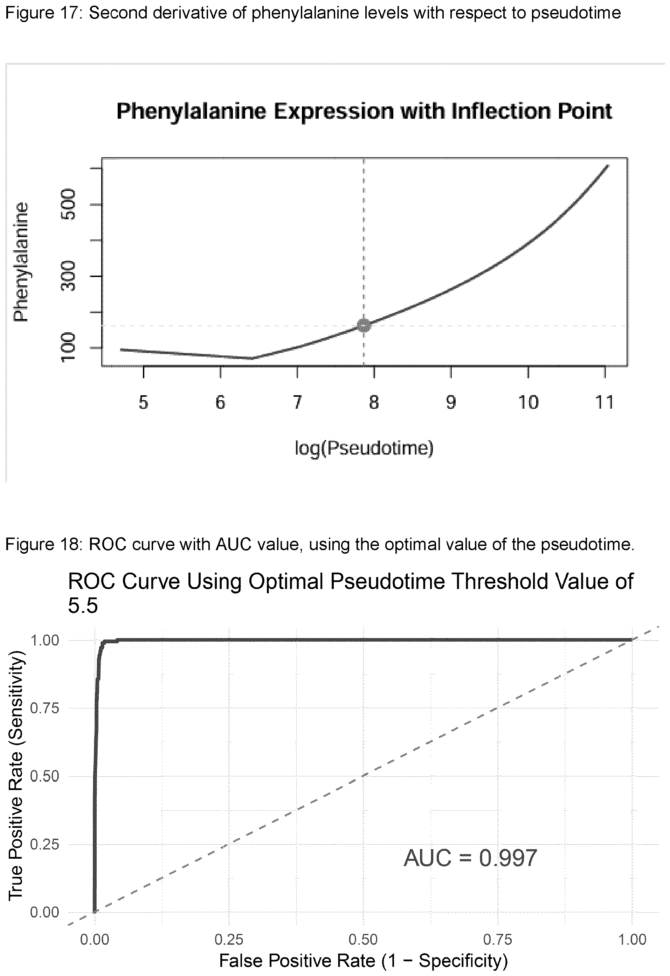

As illustrated in the accompanying examples and figures, phenylalanine is the most effective predictor of diabetes (see example 9, figures 8 to 17). In particular, specific threshold values are indicative of diabetes, including the status or stage of diabetes.

Therefore, the present invention refers to a method for the diagnosis, prognosis or prediction of diabetes mellitus comprising

> providing a biological sample of a subject and determining the amount of phenylalanine,
> wherein a threshold value of less than 77 - 83 nmol/ml, in particular less than 81 nmol/ml is indicative for a non-diabetic status,
> and/or
> wherein a threshold value of 81 - 105 nmol/ml is indicative for a pre-diabetic status, and/or
> wherein a threshold value of more than 105 - 162 nmol/ml is indicative for a moderate diabetic status,

and/or
wherein a threshold value of more than 162 nmol/ml is indicative for an advanced diabetic status,
wherein all mentioned values may deviate by +/- 10 nmol/m, +/- 5 nmol/m or +/- 2 nmol/m.

A "non-diabetic status" as used herein refers to a state where an individual does not meet the diagnostic criteria for diabetes mellitus. It indicates normal blood glucose levels and the absence of impaired glucose metabolism. This status is typically determined through clinical measures such as fasting blood glucose, glycated hemoglobin (HbA1c), or glucose tolerance testing.

A "pre-diabetic status" as used herein refers to a condition where blood glucose levels are higher than normal but not high enough to meet the diagnostic criteria for diabetes. It indicates impaired glucose metabolism and represents a risk factor for developing T2D and cardiovascular disease. Pre-diabetes is a critical stage for intervention to prevent or delay the progression to diabetes. This status is usually a early stage of diabetes.

A "moderate diabetic status" as used herein generally refers to a condition where diabetes has been diagnosed, but glucose levels are moderately elevated and are either controlled with some intervention or not yet leading to severe complications. Individuals in this status often require medical and lifestyle management but may not exhibit advanced symptoms or organ damage associated with poorly controlled diabetes. A subject is at risk.

An "advanced diabetic status" as used herein refers to a stage of diabetes where the condition is poorly controlled or has progressed to cause significant health complications. This stage is characterized by consistently high blood glucose levels, long-standing disease, and the presence of acute or chronic complications affecting multiple organ systems. Advanced diabetes often requires intensive medical management to mitigate further damage and improve the quality of life. A subject is at high or severe risk.

A "threshold value" or "cut-off value" as used herein is a predefined numerical limit used to make decisions or categorize data. When a value crosses the threshold, it triggers a specific action, classification, or outcome.

In a preferred embodiment of the invention the above mentioned diagnosis takes place for prognosis, for prophylaxis, for early detection and detection by means of differential diagnosis, for assessment of the degree of severity, and for assessing the course of diabetes mellitus, particularly Type II diabetes mellitus, and its concomitant illnesses and sequelae, as an accompaniment to therapy, and for assessing clinical decisions, particularly further treatment by means of medications for the treatment or therapy of diabetes mellitus, particularly Type II diabetes mellitus, and its concomitant illnesses and sequelae.

It is understood that aspects of the embodiments described here which have been described in the context of a device also represent a description of a corresponding method. Some or all of the method steps may be performed by (or using) a hardware device, such as a processor, a microprocessor, a programmable computer, or an electronic circuit. In some embodiments, one or more of the key method steps may be performed by such a device.

Embodiments of the invention may be implemented in hardware and/or software. Therefore, the digital storage medium can be computer-readable. Some embodiments according to the invention comprise a data carrier with electronically readable control signals that can interact with a programmable computer system so that one of the methods described herein is carried out.

In general, embodiments of the present invention may be implemented as a computer program product having a program code, wherein the program code is effective for carrying out one of the methods when the computer program product is running on a computer. The program code can, for example, be stored on a machine-readable medium. Further embodiments include the computer program for carrying out one of the methods described herein, which is stored on a machine-readable carrier.

Another embodiment of the present invention is a storage medium (or a data carrier or a computer-readable medium) having a computer program for carrying out any of the methods described herein when executed by a processor. The data carrier, the digital storage medium or the recorded medium is usually tangible and/or not seam less.

Another embodiment of the present invention is an apparatus as described herein comprising a processor and the storage medium.

Another embodiment of the invention is a data stream or a signal sequence representing the computer program for carrying out one of the methods described herein. For example, the data stream or signal sequence can be configured to be transmitted over a data communications connection, for example over the Internet.

Another embodiment includes a processing means, for example a computer or a programmable logic device, configured or adapted to perform any of the methods described herein.

A further embodiment comprises a computer on which the computer program for carrying out one of the methods described herein is installed.

Another embodiment according to the invention comprises a device or system configured to transmit a computer program for carrying out one of the methods described herein to a recipient. The receiver may be, for example, a computer, a mobile device, a storage device or the like. The device or system may, for example, comprise a file server for transmitting the computer program to the recipient.

If a term is labelled with an indefinite or definite article, such as "a" in the singular, this also includes the term in the plural and vice versa, unless the context clearly states otherwise.

The term "comprising" as used herein not only includes the meaning of "containing", but can also mean "consisting of" and "consisting essentially of".

Examples and figures:

The invention is further illustrated by the following examples and figures, without limiting the invention to these embodiments.

Example 1:

Sample collection and preparation

Blood was collected using blood collection kits for laypersons (cerascreen GmbH, Schwerin, Germany) according to the manufacturer's instructions. After disinfection of the fingertip, capillary blood samples were obtained using an automated lancet (BD Biosciences, Berkshire, UK). For each user, at least two 12 mm circles on the Dried Blood Spot Card (Ahlstrom, Bärenstein, Germany) were filled with capillary blood. The cards were air dried for at least 2 hours and sent to the laboratory by standard mail.

An isotope dilution method using high performance liquid chromatography coupled to tandem mass spectrometry (ID-LC-MS/MS) is used to determine the amino acid profile. Free amino acids are analysed, including Ala, Arg, Asn, Asp, Gln, Glu, Gly, His, Ile, Leu, Lys, Met, Phe, Pro, Ser, Thr, Trp, Tyr, Val, Cit, hArg, Orn, Sarc, bAla, Tau and GABA. A combination of protein precipitation and derivatisation is used to prepare the dried blood spot sample (2x3mm disc).

Amino acid extraction with methanol:water (4:1) with 0. 1N hydrochloric acid followed by addition of isotopically labelled internal standards (13C5-Cit, 13C7-hArg, 13C5-Orn, 13C3-Sar, 13C3-$\beta$-Alanine, 13C3-Ala, 13C6-Arg, 13C4-Asp, 13C5-Glu, 13C2-Gly, 13C6-His, 13C6-Ile, 13C6-Leu, 13C6-Lys, 13C5-Met, 13C9-Phe, 13C5-Pro, 13C3-Ser, 13C4-Thr, 13C9-Tyr, 13C5-Val, 13C5-Glu, 13C4-Asn, 13C11-Trp, 2H4-Tau, 2H6-GABA). The extraction was carried out at room temperature for 15 min at a shaking speed of 450 rpm. After centrifugation of the extract (30 $\mu$l), the transferred supernatant was evaporated in a nitrogen stream (12 l/min, 15 min at 55°C). Derivatisation by the n-butylation reaction is carried out by adding (25 $\mu$l), n-butanol in 3N hydrochloric acid to the dry residue and allowing the reaction to run for 25 min (60°C, 450 rpm). After evaporation, the dry residue is dissolved in a methanol:water mixture (1:9) with 0.1% formic acid and analysed by LC-MS/MS. The chromatographic separation of the amino acids is carried out on a thermostatted column (40°C) in reversed phase mode (Agilent, Zorbax Eclipse XDB-C18, 80Å, 4,6 x 50 mm, 1,8 $\mu$m) at a flow rate of 0,8 ml/min in a linear gradient (from 97 %:3 % to 5 %:95 %) of water and acetonitrile:methanol (1:1), both with 0,1 % formic acid as mobile phase.

Analysis is performed on a Shimadzu NexeraXR LC-20AD XR liquid chromatograph using a CTC PALxt autosampler coupled to a Sciex QTRAP4500 tandem mass spectrometer equipped with an electrospray ion source (TurboV ion source). Ions are observed in positive ion mode at 600°C with electrospray generated at 4500 V and nebuliser (GS1) and desiccant (GS2) gas flows of 45 psi and 50 psi respectively. Unique fragmentation reactions are observed by single reaction monitoring. Quantitative analysis by isotope dilution is performed using a 7-point calibration curve generated in solvents. The method has satisfactory sensitivity at a limit of quantification (LOQ) and adequate analytical parameters (varying between analytes) including linearity (R2 greater than 0.995), recovery (85-98%), precision (less than 10%) and accuracy (relative bias less than 15%).

Example 2:

Data collection

The dataset is retrieved from the applicant's mycerascreen database and contains 7290 observations and 69 characteristics. These characteristics represent 26 amino acids and amino acid metabolites, information on 8 diet types, 33 symptoms and diseases, age, height, weight and sex. All observations are from customers of cerascreen GmbH who have taken the cerascreen amino acid test and provided personal and health information via the app regarding diseases or symptoms they were experiencing at the time of the test. The dataset includes individuals between the ages of 18 and 93, all of whom consented to the use of their data for research purposes. Of the total population, 175 people (2.4%) are diabetic.

Example 3:

Data preprocessing

Except for amino acid measurements and age, all other data in the dataset are binary, where 0 represents negative and 1 represents positive for each condition. Only observations for which we have no missing data (7290) are used for further analysis. This includes 175 diabetics.

Deriving the adjusted amino acid values for each amino acid

One of the main challenges in using observational (non-randomised) data is that the treatment (i.e. the diabetic) and control (non-diabetic) groups are not balanced at baseline. In other words, the two groups may differ in important ways (such as diet, age, disease or symptom) that can effect both amino acid levels and the likelihood of developing diabetes. Without accounting these these baseline differences, it is difficult to isolate the effect of amino acid levels on diabetes progression. To address this, we apply propensity score weighting causal analysis to perform in silico randomisation of the dataset to adjust amino acid amounts (levels) for each amino acid. Before performing the causal analysis for each amino acid (outcome), we need to filter the dataset for highly correlated independent variables.

Filtering by correlation

To fit the amino acid amounts (levels) for each amino acid to all other features in the data set, each amino acid is treated as an outcome and all other features are treated as predictors. Using the *recipe()* function from the recipes R package (Kuhn M, Wickham H, Hvitfeldt E (2024). recipes: Preprocessing and Feature Engineering Steps for Modeling. R package version 1.1.0, https://recipes.tidymodels.org/, https://github.com/tidymodels/recipes.), highly correlated predictors are filtered out using a Pearson correlation coefficient threshold of 0.5. If two predictors have a correlation coefficient greater than 0.5 (either positive or negative), one of them is removed from the model based on how it correlates with other features in the data. This means that different sets of covariates are filtered out depending on the amino acid in question. After this step, the numeric binary variables in the dataset are binarised and Inverse Probability of Treatment Weighting (IPTW) is applied.

Propensity score weighting.

The propensity score $e(X)$ is the probability of being diabetic given the observed covariates.

$$e(X) \ = \ P(T \ = \ 1 \,|\, X)$$

Where T = 1 represents presence of diabetes

IPTW assigns weights based on the inverse of the propensity score, $\frac{1}{e(X)}$, for the diabetic group and $\frac{1}{1-e(X)}$ for the non-diabetic group.

Propensity score weighting is a technique used to create a pseudo-population in which the covariates in the dataset are balanced between the non-diabetic and diabetic groups, making the groups comparable as if the data came from a randomised controlled trial (Chesnaye NC, Stel VS, Tripepi G, Dekker FW, Fu EL, Zoccali C, Jager KJ. An introduction to inverse probability of treatment weighting in observational research. Clin Kidney J. 2021 Aug 26;15(1):14-20. doi: 10.1093/ckj/sfab158. PMID: 35035932; PMCID: PMC8757413). Inverse Probability of Treatment Weighting (IPTW) assigns weights to each subject (individual) based on their propensity score p, which is the probability that the individual is diabetic or not. The aim is to estimate the Average Treatment Effect on the Treated (ATT), which allows the effect of diabetes to be estimated in diabetic individuals compared to non-diabetics with similar covariates. By assigning weights, subjects who look like the opposite group (e.g. non-diabetics who have similar metabolic profiles to diabetics) are given higher weights because they serve as better counterfactuals. This balances the covariate between the diabetic and non-diabetic groups. See Table 1. below for a better understanding.

Table 1. Example table with Weights for ATT:

| Group | Propensity Score | Weight |
|---|---|---|
| Diabetic | 0.1 | 1.0 |
| Diabetic | 0.5 | 1.0 |
| Diabetic | 0.9 | 1.0 |
| Non-diabetic | 0.1 | 0.11 |

(continued)

| Group | Propensity Score | Weight |
|---|---|---|
| Non-diabetic | 0.5 | 1.0 |
| Non-diabetic | 0.9 | 9.0 |

Diabetic individuals are all weighted equally (weight = 1) because ATT focuses on estimating the effect of treatment for the treated group (here, the diabetic group). Non-diabetic individuals are weighted based on their propensity score p, with weights calculated as $\frac{p}{(1-p)}$ This means that non-diabetics who have a higher propensity score (i.e., who look similar to diabetics) are given higher weights.

For example, a non-diabetic individual with a high propensity score of 0.9 (i.e., they look like a diabetic) gets a high weight of 9.0 because they serve as a better counterfactual for the diabetic group.

Conversely, a non-diabetic with a low propensity score (0.1) gets a smaller weight of 0.11 because they do not resemble the diabetic group as much.

Visualizing the distributional balance of propensity scores

The process of generating these weights is iterative.

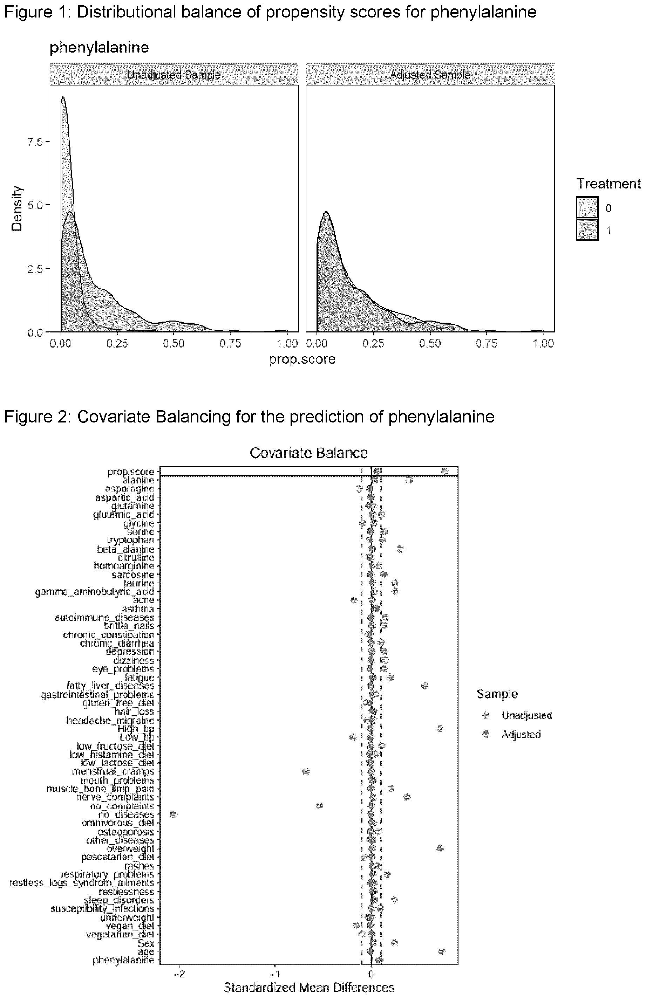

Figure 1 shows how to assess the effectiveness of IPTW for phenylalanine prediction.

Conclusion:

Figure 2 shows that the preprocessing or adjustment procedure has improved the balance for most covariates, making the diabetic and non-diabetic groups more comparable in terms of these variables. Covariates that were previously unbalanced (yellow points far from 0) are now closer to balance (green points near 0). By achieving covariate balance, any subsequent analysis (e.g. estimating the effect of diabetes on amino acid levels) will be less biased by confounding factors, leading to more reliable causal inferences.

Inverse probability of treatment-weighted linear regression modelling

A weighted linear regression model is fitted for each amino acid to account for the imbalance between diabetics and non-diabetics. The outcome is the amino acid in question (e.g. phenylalanine), the predictors are the main effects of diabetes and other covariates on that amino acid and the interaction effect of diabetes and all other covariates. The interaction term captures how diabetes may affect the outcome differently depending on the levels of other covariates and thus provides better estimates. The corrected values of amino acid levels are extracted by taking the absolute values of the predictions (fitted values). The results are the adjusted amino acid levels, adjusted for any imbalances between the diabetic and non-diabetic groups.

This method uses weights from IPTW to fit the regression model. The weights adjust for the influence of each data point to correct for the unbalanced sample.

For a dataset with n observations, where each observation has a weight w, the weighted least squares estimator for the regression coefficients $B = (X^TWX)^1 X^TWy$ wherein:

> X is the matrix of predictors
> y is the vector of the observed response, the amino acid in question
> W is a diagonal matrix of IPTW $w_i$ where W = diag $(w_1, w_2, ..., w_n)$
> $X^T$ is the transpose of X
> $(X^TWX)^1$ is the inverse of the weighted normal equations
> How it works

The weights $w_i$ are inversely proportional to the variance of the observations. They are the IPTW which means that these weights come from the propensity scores calculated for each observation.

The idea behind the weighted least squares is that observations with higher variance (less reliable) receive lower weights, and those with lower variance (more reliable) receive higher weights, thereby improving the efficiency of the estimates.

Example 4:

Calculation of the pseudotime

Subsetting the data for non diabetic individuals

Amino acid data from non-diabetic observations are subsetted and used for the calculation of the Mahalanobis distance.

Calculating of the Robust Covariance Matrix and Mean using covMcd:

The Minimum Covariance Determinant (MCD) method, implemented by the *covMcd*() function, computes the robust mean (center) and robust covariance matrix. This is useful in accounting for outliers, as the MCD finds the subset of data that minimizes the determinant of the covariance matrix and uses it to compute robust estimates.

Robust mean: The robust center or mean of the non-diabetic subjects.

Robust covariance matrix: the robust covariance matrix of the non-diabetic individuals. Calculating the Mahalanobis Distances for all subjects:

This part calculates Mahalanobis distances for all subjects, i.e. diabetic and non-diabetic in the data set using the robust center and covariance matrix derived from the non-diabetic individuals.

Mahalanobis Distance: This distance metro accounts for the correlations between variables (in this case, amino acids) and scales the distances by the covariance matrix, and it is calculated as:

$$D_M(x) = \sqrt{(x-\mu)^T \Sigma^{-1}(x-\mu)}$$

Where:

$x$ is the data point (amino acid levels)
$\mu$ is the robust mean of the corrected amino acid levels in the diabetics
$\Sigma$ ist he robust covariance matrix

Using the Mahalanobis Distance as the pseudotime:

Mahalanobis distances are used as a preferred method for calculating the pseudotime. In this context, the pseudotime represents the "distance from the root centroid" or how far each individual's amino acid profile is from the robust centre of the non-diabetic group.

The aim is to calculate these distances and use them as a measure of pseudotime that reasonably represents metabolic progression. The Minimum Covariance Determinant (MCD) to calculate a robust mean and robust covariance matrix for the non-diabetic subjects. Robust Mahalanobis Distance is used to assess how far each subject (both diabetic and non-diabetic) deviates from the typical profile of non-diabetic subjects. The use of robust MCD ensures that outliers (which could bias the results) do not unduly influence the mean and covariance estimates.

Individuals further from the typical non-diabetic profile (i.e. those with higher Mahalanobis distances) can be interpreted as being further along a metabolic trajectory that may indicate progression to diabetes or more severe diabetes.

Example 5:

Determining the optimal log pseudotime threshold to classify diabetic state

The inventors performed a threshold-based classification to predict diabetes status. Specifically, we tested a series of thresholds for log-transformed pseudotime values. A patient was classified as Diabetic if their log-pseudotime exceeded the threshold and Non-diabetic otherwise.

The sequence of thresholds ranged from the minimum to the maximum observed log-pseudotime in increments of 0.5. For each threshold, predictions were generated using the following rule:

$$predicted = \begin{cases} Diabetic & if \log(pseudotime) > threshold \\ Non-diabetic & otherwise \end{cases}$$

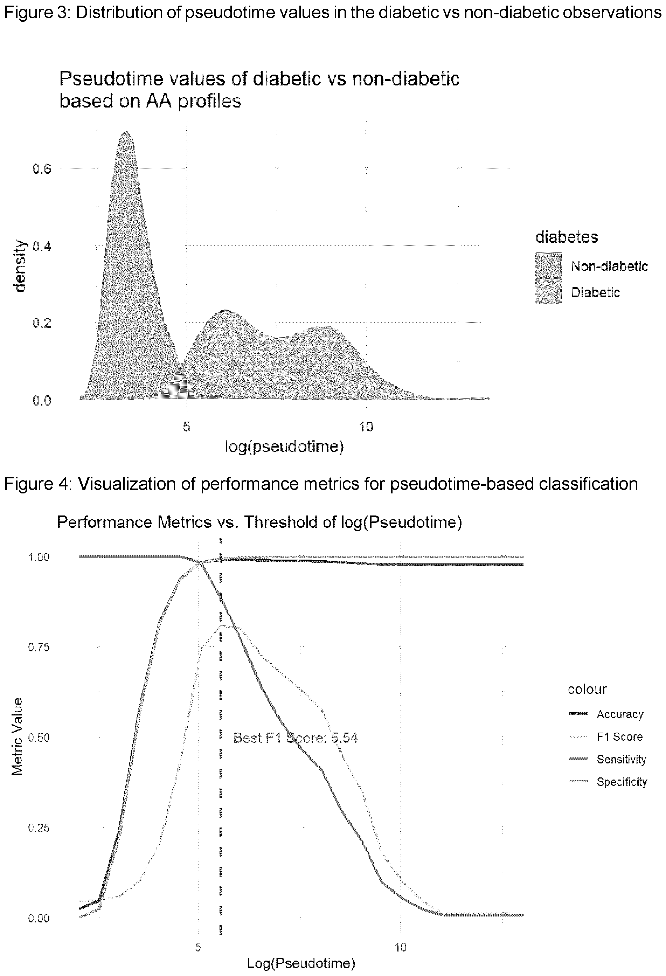

The results are shown in Figures 4 to 7.

Example 6:

Random forest regression for predicting log-pseudotime

The inventors employed a Random Forest regression model to predict log-transformed pseudotime, which represents the metabolic trajectory of diabetes progression, using a combination of corrected amino acid levels, demographic factors (age, sex, BMI), and diabetes status as predictor variables. The Random Forest algorithm was chosen for its ability to model complex, non-linear relationships without requiring strict assumptions about the distribution of the data.

The model used amino acid amounts (levels) (alanine, phenylalanine, citrulline, etc.), alongside demographic factors (features) such as age, sex, BMI, and diabetes status (binary: diabetic or non-diabetic) to predict log-pseudotime. These variables were selected based on their known or hypothesized associations with metabolic changes and diabetes risk.

The response variable was the log-transformed pseudotime value, which was derived from a Mahalanobis distance metric applied to the corrected amino acid profiles. The log transformation was applied to stabilize variance and reduce skewness in the distribution of pseudotime values.

The inventors used the *randomForest* () function in R to implement the Random Forest model. The number of trees was set to 500 to ensure stable predictions, and the default value of randomly selected predictors at each split (mtry) was used.

The importance of each feature in predicting the log pseudotime is shown in Figure 8 below.

Example 7:

Random Forest Model for Predicting Diabetes

A Random Forest (RF) model was trained to predict the occurrence of diabetes based on log-transformed pseudotime, phenylalanine levels, BMI, age, and sex. log-transformed pseudotime, phenylalanine levels were particularly selected because they appeared in our previous analysis as the features that best predict pseudotime progression. The following steps were undertaken for model training and evaluation: Data Preprocessing and Variable Selection:
The dataset was split into training (80%, 5825 observations) and test sets (20%, 1455 observations). Key predictors used for model development included log_pseudotime, phenylalanine, BMI, age, and Sex. The response variable was diabetes status (Diabetic/Non-diabetic).

Cross-validation

To optimize the model's performance, 5-fold cross-validation was employed. The cross-validation strategy was defined using the *trainControl*( ) function from the caret package, specifying the use of 5-fold cross-validation (number = 5) with grid search (search = "grid") for hyperparameter tuning. This approach ensured that the model was evaluated on different subsets of the data, minimizing overfitting and improving generalizability.

Hyperparameter Tuning:

A grid search was conducted over the tuning parameter mtry (the number of predictors sampled at each split in the RF). The values tested were 2, 3, 4, and 5, which provided a range of complexity for the Random Forest model.

The tuning grid was defined using *expand. grid*(*mtry* = c(2, 3, 4, 5)).

Model Training:

The RF model was trained using the *train*( ) function, where the cross-validation (trControl) and tuning grid (tuneGrid) were specified. The model was trained on the training dataset with the predictors: log_pseudotime, phenylalanine, BMI, age, and Sex.

A seed was set (set.seed(123)) for reproducibility to ensure consistent results across different runs.

Model Selection:

The optimal value of mtry was determined from the cross-validation results (*rf_model_cv$bestTune*). This optimal parameter was then used to fit the Final Random Forest model, which was trained with 500 trees (*rtree* = 500).

Variable Importance:

The importance of each predictor was assessed using the *varImpPlot*( ) function from the random Forest package, providing insights into which predictors were most influential in determining diabetes status. This visualizes the contribution of each predictor to the model's decision-making process. The graph below shows the feature importance from the best-performing RF model trained to predict diabetes status using variables such as log-transformed pseudotime, phenylalanine levels, BMI, age, and sex.

Model prediction:

The trained RF model was applied to the test dataset to predict diabetes status. Predictions were generated using the *predict*( ) function, and these predictions were compared against the actual labels.

Model performance evaluation:

A confusion matrix was generated using the *confusionMatrix*( ) function to evaluate the model's accuracy, sensitivity, specificity, positive predictive value (PPV), and negative predictive value (NPV). These metrics were calculated to assess the classification performance on the test set.

Model saving:

The trained Random Forest model was saved as an RDS file for future use, ensuring reproducibility and enabling further application to unseen data.

Significance of the Methodology:

The use of Random Forest with cross-validation ensures robust model performance by preventing overfitting and selecting the optimal complexity for the model. The use of hyperparameter tuning via grid search for mtry enhances the model's generalizability to new data. Moreover, the inclusion of biologically relevant features such as phenylalanine and BMI provides a biologically interpretable model for predicting diabetes.

This methodology allows for an in-depth evaluation of the model's performance across multiple metrics, ensuring that the model is both accurate and practical for real-world applications.

The RF model was evaluated on a validation set comprising 565 observations. The amino acid concentrations were corrected using the same models as for the training data and the pseuodtime calculated using the same robust mean and robust covariate values. The confusion matrix yielded an overall accuracy of 99.47%, with a 95% confidence interval (CI) of 98.46% to 99.89%. The model's accuracy was significantly higher than the No Information Rate (NIR) (97.35%), as evidenced by a highly significant p-value of 0.0001852 (P-value [Acc > NIR] < 0.001).

The kappa statistic, which measures the agreement between predicted and true classes beyond chance, was 0.8938, indicating strong agreement. Sensitivity (True Positive Rate) was recorded at 86.67%, indicating that the model correctly identified 86.67% of the actual diabetic cases in the validation data. Specificity (True Negative Rate) was exceptionally high at 99.82%, demonstrating the model's ability to accurately classify non-diabetic individuals. The model's positive predictive value (PPV), which reflects the proportion of predicted diabetic individuals that are truly diabetic, was 92.86%. Meanwhile, the negative predictive value (NPV) was 99.64%, ensuring that nearly all non-diabetic predictions were correct.

The prevalence of diabetes in the validation dataset was 2.65%, and the model's detection rate (Recall or Sensitivity) for diabetic cases was 2.30%. The model's detection prevalence was 2.48%, indicating that the model flagged 2.48% of the individuals as diabetic. The balanced accuracy, which takes into account both sensitivity and specificity, was 93.24%, indicating the model's strong overall performance across both classes.

McNemar's test p-value was 1.0, signifying no statistically significant differences between the false positives and false negatives, which further supports the reliability of the predictions.

Example 8:

Metabolic progression of phenylalanine from non-diabetic to diabetic state A LOESS smooth line was fitted to help observe the general trend of the corrected phenylalanine values as pseudotime increased, providing insight into how phenylalanine levels may change along the metabolic trajectory. The plot allows the exploration of how phenylalanine values change across pseudotime for both diabetic and non-diabetic individuals. The red and blue data points represent phenylalanine expression, categorized by a diabetic state, with the blue and red points clustered differently. This supports the hypothesis that higher pseudotime values correlate with increased phenylalanine expression, potentially distinguishing between diabetic and non-diabetic individuals.

Example 9:

Determination of diabetes-relevant thresholds of phenylalanine

Phenylalanine threshold relevant for diabetic classification

Procedure:

Empty lists are created to store values for accuracy, sensitivity, specificity, precision, and F1 scores across different thresholds.

The code iterates over a specified range of threshold values ('threshold_range'), which define the cutoff levels of phenylalanine to classify a sample as diabetic or non-diabetic.

For each threshold:

Samples are classified as "Diabetic" if the phenylalanine level exceeds the threshold, and " non-diabetic" otherwise.

A confusion matrix is generated to assess the classification performance for each threshold. This matrix helps calculate various performance metrics.

The code extracts precision (positive predictive value), sensitivity (recall), and specificity from the confusion matrix.

The F1 score is calculated based on precision and recall to provide a balanced measure of performance.

Each metric-accuracy, sensitivity, specificity, precision, and F1 score-is stored in its respective list for further analysis.

Phenylalanine threshold relevant for pre-diabetes

In this process, we calculate and visualize the density distributions of log-transformed phenylalanine levels in diabetic and non-diabetic individuals. This analysis aims to identify a phenylalanine threshold that maximizes classification accuracy by balancing sensitivity and specificity in diagnosing diabetes.

Procedure:

Calculating prior probabilities for diabetic and non-diabetic groups.

Computing the mean and standard deviation of phenylalanine levels for both groups. Defining density functions for each group and a mixture density function combining them.

Visualizing the mixture density, diabetic density, and non-diabetic density curves. Highlighting the overlap region where the distributions of the two groups intersect. Drawing a vertical threshold line indicating a potential cutoff for classifying individuals as diabetic or non-diabetic based on phenylalanine levels.

Transition zone to diabetes based on phenylalanine values

To identify the phenylalanine level at which the probability of being diabetic reaches or exceeds 50% (posterior probability $\geq 0.5$), based on Bayesian analysis, we used the following procedure:

Procedure

Each phenylalanine level (from 50 to 300) is log-transformed to match the distributions for diabetics and non-diabetics.

For each log-transformed value, the code calculates the probability density of phenylalanine for diabetic and non-diabetic groups using normal distribution parameters ('mu_diabetics', 'sigma_diabetics', 'mu_non_diabetics', 'sigma_non_diabetics').

Bayes' theorem is applied with prior probabilities of diabetes (0.02) and non-diabetes (0.98) to compute the posterior probability of being diabetic for each phenylalanine level.

The code identifies the phenylalanine level at which the posterior probability of diabetes first reaches 0.5, marking this as the optimal cutoff for classifying an individual as diabetic.

The optimal phenylalanine cutoff level is printed, which is the point where an individual has a 50% or greater probability of being classified as diabetic based on phenylalanine concentration.

Phenylalanine threshold for advanced diabetes

To assess how phenylalanine levels change over pseudotime, capturing metabolic shifts that may occur along the diabetes progression. This analysis provides insight into how phenylalanine levels evolve over pseudotime, identifying regions where metabolic changes occur most prominently.

First derivative

Modeling Phenylalanine Levels: Predicted phenylalanine levels are derived based on log-transformed pseudotime using a fitted model.

The first derivative of phenylalanine levels with respect to pseudotime is computed, showing how quickly phenylalanine levels are increasing or decreasing at each pseudotime point.

Second derivative:

To explore the dynamics of phenylalanine levels across pseudotime by calculating the first and second derivatives with respect to log-transformed pseudotime. This provides insight into both the rate of change and the acceleration or deceleration of phenylalanine levels over time.

Approach of second derivative:

A model is used to predict phenylalanine levels based on log-transformed pseudotime values.

The first derivative with respect to log-transformed pseudotime quantifies the rate of change of phenylalanine levels. Positive values indicate an increase in phenylalanine, while negative values indicate a decrease.

The second derivative is calculated to assess the acceleration or deceleration of phenylalanine levels. Positive values suggest increasing rates of change (convex trend), while negative values indicate decreasing rates of change (concave trend).

Summary of phenylalanine levels, relevant for diabetes

Given the three thresholds we have calculated, (81, 105, and 162.28 nmol/ml), we can define specific phenylalanine ranges that may help categorize individuals based on their diabetes risk and stage. Here's a summary interpretation of each threshold and the corresponding risk implications:

Summary of Threshold-Based Ranges

| Phenylalanine Range (nmol/ml) | Risk Level | Interpretation |
|---|---|---|
| < 81 | Low Risk | Likely non-diabetic or well-controlled diabetic |
| 81 - 105 | Intermediate Risk / Pre-Diabetic | Early or pre-diabetic stage, partial metabolic control |
| 105 - 162.28 | High Risk | Moderate to advanced diabetes, higher risk of dysregulation |
| ≈ 162.28 | Critical Transition Point | Key metabolic shift, onset of severe complications |
| > 162.28 | Very High Risk | Poorly controlled or advanced diabetes, severe risk |

Individuals in the 81-105 nmol/ml range may benefit from lifestyle changes and early interventions to prevent progression to diabetes. For those in the 105-162.28 nmol/ml range, more focused glucose management and frequent monitoring are recommended to avoid metabolic complications. Patients with phenylalanine levels above 162.28 nmol/ml may require aggressive treatment and monitoring due to the high likelihood of advanced diabetes and associated complications.

Figure 1:

Preprocessing (=adjusting)

Unadjusted sample (left panel):

This panel shows the density distribution of propensity scores before any adjustment (e.g., before applying propensity score weighting).
Yellow (0) represents individuals in the non-diabetic group.
Green (1) represents individuals in the diabetic group.
We can see that the two groups have very different distributions of propensity scores, indicating that the groups are not balanced in terms of the covariates used to calculate the propensity score. The non-diabetic group has mostly low propensity scores (closer to 0), whereas the diabetic group has a broader distribution, with many individuals having higher propensity scores.

Adjusted sample (right panel):

This panel shows the density distribution of the propensity scores after adjustment (by IPTW).
After adjustment, the propensity score distributions of the diabetic and non-diabetic groups are much more similar. The overlap between the groups has increased, indicating that the covariates are now more balanced between the two groups.
The adjustment helps to ensure that the groups are comparable, allowing a more valid estimate of the causal effect of having diabetes on lysine levels.

Summary:

Unadjusted sample: The distributions are different, meaning that the covariates differ between the two groups. Without adjustment, any comparison between diabetics and non-diabetics may be biased because the groups are not comparable.
Adjusted sample: The distributions are more similar, meaning that the covariates have been balanced between the two groups. After this adjustment, comparisons between groups are more reliable and better reflect the causal relationship.
Figure 2 is a covariate balance plot, which compares the standardized mean differences of covariates between the diabetic and non-diabetic groups both before (unadjusted) and after (adjusted and preprocessed) the application of propensity score weighting.

Understanding the Plot:

X-Axis shows the standardized mean differences (SMD) between the treatment and control groups for each covariate.
The further a point is from 0, the more unbalanced that covariate is between the treatment and control groups.
Values close to 0 indicate balance between the groups.
Y-Axis lists the covariates that were included in the propensity score model or balancing procedure. This includes:

Amino acids: such as lysine, serine, glutamine, etc.
Health conditions: such as asthma, fatigue, depression, etc.
Diet and lifestyle factors: such as gluten-free diet, vegan diet, etc.
Demographic variables: such as age and sex.

Points:

Yellow (Unadjusted): These points show the standardized mean differences before adjustment, where we expect greater imbalances between covariates in the diabetic and non-diabetic groups.
Green (Adjusted): These points represent the adjusted standardized mean differences, after applying propensity score weighting (or another method). The green points should generally be closer to 0, showing that covariates between groups are more balanced.

Dotted Vertical Lines: These lines typically indicate a threshold for balance (e.g., SMD = ±0.1). Points within this range (closer to 0) are considered balanced, while those outside indicate imbalance.

Summary:

Unadjusted Sample (Yellow):

You can see several covariates (e.g., amino acids, certain health conditions) have large standardized mean differences before adjustment. This indicates that these covariates are not balanced between the diabetic and non-diabetic groups in the original data.
For example, some points (such as for certain dietary conditions) are far from 0, indicating major imbalances between groups.

Adjusted Sample (Green):

After adjustment, most of the green points are closer to 0, indicating improved balance across most covariates. This shows that the adjustment using IPTW has successfully reduced the imbalance between the diabetic and non-diabetic groups in predicting phenylalanine.
However, some covariates might still have some residual imbalance, as indicated by green points further from 0 (though still improved compared to the unadjusted sample).

Figure 3:

A logarithmic transformation is applied to reduce skewness and better visualize the distribution of the pseudotime in the diabetic and non-diabetic groups.
This density plot compares the log-transformed pseudotime values between diabetic and non-diabetic groups based on their amino acid profiles. The plot suggests that pseudotime is a strong discriminatory feature for distinguishing diabetics from non-diabetics. This makes pseudotime a potentially useful biomarker or measure for assessing metabolic health or progression towards diabetes.

Non-diabetic Group (Red Curve):

The distribution is highly concentrated at lower pseudotime values, indicating that non-diabetic individuals tend to have metabolic profiles closer to the baseline (which is expected, as pseudotime represents metabolic progression).
There is a sharp peak around log(pseudotime) ~ 3, showing that most non-diabetics have relatively low pseudotime values.

Diabetic Group (Teal Curve):

The distribution of pseudotime for diabetic individuals is spread across higher pseudotime values compared to non-diabetics.
The curve has multiple peaks, which could indicate subgroups within the diabetic population or heterogeneity in how diabetes affects metabolic progression.

Interpretations:

Separation between the groups: The clear separation between the distributions suggests that diabetics generally have higher pseudotime values, reflecting more significant metabolic progression or deviation from the healthy (non-diabetic) baseline.
Multiple peaks in diabetics: The broader and more complex shape of the diabetic distribution might imply that diabetes affects different individuals to varying extents, perhaps due to factors like disease severity, duration of diabetes, or the presence of other metabolic conditions.

Figure 4:

The graph shows the relationship between various performance metrics-accuracy, F1 score, sensitivity, and specificity-and different threshold values of the log-transformed pseudotime. It evaluates the performance of a predictive model at various thresholds to determine an optimal cutoff for predicting diabetes.

Description:

X-axis (Log [Pseudotime]): Represents the range of log-transformed pseudotime values tested as thresholds.

Y-axis (Metric Value): Indicates the value of each performance metric (ranging from 0 to 1).

Curves:

Accuracy (blue): Remains near 1 across most threshold values, indicating consistently high accuracy.

F1 Score (green): Peaks at a specific pseudotime threshold (~5.54), highlighting the best trade-off between precision and recall at this point.

Sensitivity (red): Measures the model's ability to correctly identify diabetic cases (true positives). It decreases after the optimal threshold, indicating a drop in true positives. Specificity (orange): Remains high across thresholds, indicating the model's ability to correctly identify non-diabetic cases (true negatives).

Interpretation:

The optimal threshold for predicting diabetes is around log(pseudotime) = 5.54, where the F1 score is highest. This suggests that this threshold offers the best balance between precision and sensitivity, making it an effective choice for classifying diabetic vs. non-diabetic patients.

Summary:

The model achieves consistently high accuracy and specificity across most thresholds.

Sensitivity decreases sharply as thresholds increase, which could result in more false negatives.

The F1 score peaks at the threshold value of 5.54, indicating this point as the best trade-off for prediction accuracy in terms of both precision and recall.

This threshold can be leveraged in future applications for precise diabetes prediction, balancing the rates of false positives and false negatives effectively.

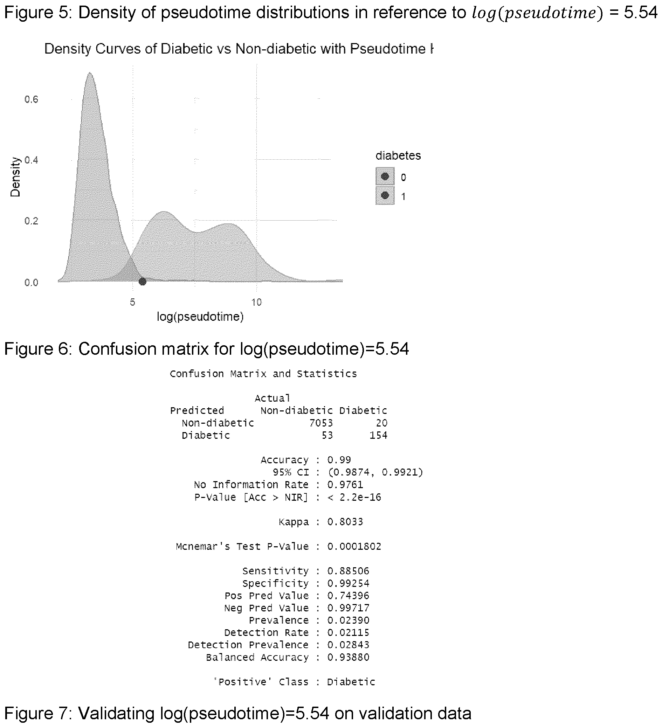

Figure 5 refers to the testing of log pseudotime threshold on the training data and shows the density distributions of *log*(*pseudotime*) for diabetic and non-diabetic individuals, with the addition of a visual marker at *log*(*pseudotime*) = 5.54. This threshold was identified as optimal based on F1 score analysis and is used here to understand its placement relative to the density distributions of the two groups.

Interpretation:

Non-diabetic distribution (cyan): The majority of non-diabetic individuals are concentrated around lower log(pseudotime) values, with a peak between 3 and 6. This suggests that individuals with lower pseudotime values are more likely to be non-diabetic.

Diabetic distribution (red): The diabetic group exhibits a broader distribution, with a significant density shift towards higher log(pseudotime) values, particularly between 8 and 10. This implies that higher pseudotime values are indicative of diabetes.

The log(pseudotime) threshold of 5.54 (depicted by the blue dot) falls near the tail of the non-diabetic distribution and at the left edge of the diabetic curve. This position suggests that a pseudotime value of 5.54 is at a critical transition point where the likelihood of classification shifts from non-diabetic to diabetic. In clinical practice, such a threshold could be useful for differentiating between the two groups, but its predictive capacity is likely improved when combined with other biological markers. Conclusion: This analysis underscores the utility of pseudotime as a predictor of diabetes.

Figure 6 relates to the classification performance of log(pseudotime)=5.54 and shows the confusion matrix, which provides a comprehensive evaluation of the model's performance for diabetes prediction. The model, evaluated on one dataset, shows an overall accuracy of 99% (95% CI: 0.9874, 0.9921), indicating a strong ability to correctly classify both diabetic and non-diabetic individuals.

Sensitivity: The model achieved a sensitivity of 88.5%, reflecting its capability to correctly identify 88.5% of diabetic cases. This is crucial for diagnostic models, as missing true positives can lead to delayed treatment.

Specificity: With a specificity of 99.25%, the model effectively classifies 99.25% of non-diabetic individuals, minimizing false positives and thus ensuring that non-diabetic individuals are not erroneously classified as diabetic.

Positive Predictive Value (PPV): The model's PPV, at 74.4%, indicates that when diabetes is predicted, there is a 74.4% chance that the prediction is correct. While relatively high, this metric suggests room for improvement in minimizing false positives.

Negative Predictive Value (NPV): The NPV of 99.77% is particularly robust, suggesting that when the model predicts a non-diabetic status, it is almost always correct.

The McNemar's Test yielded a statistically significant result (p < 0.0002), suggesting a potential bias in the model's predictions that could merit further investigation.

Conclusion:

Despite these areas for improvement, the model's strong sensitivity and balanced accuracy of 93.88% make it a promising tool for early diabetes screening. Its incorporation of both clinical (BMI, age, sex) and biochemical markers (amino acid profiles (patterns), pseudotime) introduces a novel predictive mechanism that could complement existing diagnostic methods such as blood glucose testing.

Figure 7 relates to the validation of the log pseudotime threshold on validation data. The application of a log-transformed pseudotime threshold of 5.54 to a validation data set of 565 observations yielded very promising results, as illustrated by the confusion matrix. The amino acid values of the validation data were corrected using the same models used to correct the training data. The pseudotime was calculated using the same covariance matrix and robust mean of the healthy subjects used in the training dataset. The model achieved an overall accuracy of 99.47%, demonstrating exceptional performance in distinguishing between diabetics and non-diabetics.

Interpretation:

The pseudotime threshold demonstrated exceptional specificity and high precision, suggesting its robustness in correctly identifying non-diabetic individuals and minimizing false positives. However, the sensitivity of 86.67% indicates that while the model performs well, it could potentially be enhanced further to better capture diabetic cases, as a small proportion of true diabetics were misclassified as non-diabetic. The balanced accuracy of 93.24% provides a solid reflection of the model's overall performance, accounting for both true positives and true negatives.

A log-pseudotime threshold of 5.54, presents a viable diagnostic tool for distinguishing between diabetic and non-diabetic individuals with high accuracy and specificity. The integration of pseudotime with other covariates (such as amino acid levels and phenylalanine) provides an innovative approach to diabetes prediction. While there is potential for further optimization in terms of sensitivity, the current performance metrics suggest that this model could serve as an effective diagnostic aid, particularly in conjunction with other clinical indicators. Further research may be warranted to validate these findings across larger and more diverse populations, as well as to explore the utility of amino acid levels as biomarkers in diabetes screening.

Figure 8: Best predictors of the log-pseudotime

The bar graph highlights the top non-linear predictors of pseudotime, based on feature importance in the random forest model.

Diabetes Status and Phenylalanine Lead:

Diabetes and phenylalanine have the highest importance scores, indicating that these factors are the most influential in predicting pseudotime. This suggests that metabolic progression (captured by pseudotime) is closely tied to the presence of diabetes and elevated phenylalanine levels. Phenylalanine, in particular, is a known marker associated with metabolic dysregulation in diabetes.

Amino Acids as Key Predictors:

Other amino acids like glycine, ornithine, and glutamic acid also show significant importance, reinforcing the hypothesis that amino acid metabolism plays a crucial role in the metabolic state reflected by pseudotime. Glycine, for example, has been linked to insulin resistance and metabolic syndrome, making it a relevant marker in diabetes progression.

Potential Biomarkers:

The inclusion of amino acids such as citrulline, aspartic acid, and tryptophan suggests they might serve as additional biomarkers for monitoring the metabolic state. Their involvement in urea cycle and neurotransmitter regulation could imply deeper metabolic changes, especially in the context of diabetes.

Non-Linear Relationships:

The fact that a non-linear model (random forest) was used to reveal these relationships suggests that the effect of these variables on pseudotime is not simply additive or linear. For instance, the impact of diabetes or phenylalanine might vary across different ranges of pseudotime, pointing to more complex interactions in metabolic regulation.

Summary:

The graph emphasises the important role of both clinical and biochemical factors (especially diabetes status and amino acid levels) in shaping pseudotime. This finding could guide further studies of metabolic progression and potentially lead to improved methods for early detection of diabetes or metabolic monitoring.

The importance rankings suggest that future models and interventions targeting these specific amino acids could provide valuable insights into the early detection and management of metabolic disorders, particularly diabetes. However, we also note that metabolic rather than demographic traits are better predictors of diabetes.

Figure 9 suggests that metabolic variables, especially log-transformed pseudotime and phenylalanine, are stronger indicators of diabetes than traditional demographic factors such as age and sex. The high contribution of log_pseudotime supports the notion that pseudotime captures essential non-linear dynamics related to disease progression.

Figure 10 relates to the validation of the model for predicting diabetes.

The confusion matrix for the random forest model on the validation data provides insightful details about the model's performance.

The RF model was evaluated on a validation set comprising 565 observations. The amino acid concentrations were corrected using the same models as for the training data and the pseuodtime calculated using the same robust mean and robust covariate values. The confusion matrix yielded an overall accuracy of 99.47%, with a 95% confidence interval (CI) of 98.46% to 99.89%. The model's accuracy was significantly higher than the No Information Rate (NIR) (97.35%), as evidenced by a highly significant p-value of 0.0001852 (P-value [Acc > NIR] < 0.001).

The kappa statistic, which measures the agreement between predicted and true classes beyond chance, was 0.8938, indicating strong agreement. Sensitivity (True Positive Rate) was recorded at 86.67%, indicating that the model correctly identified 86.67% of the actual diabetic cases in the validation data. Specificity (True Negative Rate) was exceptionally high at 99.82%, demonstrating the model's ability to accurately classify non-diabetic individuals. The model's positive predictive value (PPV), which reflects the proportion of predicted diabetic individuals that are truly diabetic, was 92.86%. Meanwhile, the negative predictive value (NPV) was 99.64%, ensuring that nearly all non-diabetic predictions were correct.

The prevalence of diabetes in the validation dataset was 2.65%, and the model's detection rate (Recall or Sensitivity) for diabetic cases was 2.30%. The model's detection prevalence was 2.48%, indicating that the model flagged 2.48% of the individuals as diabetic. The balanced accuracy, which takes into account both sensitivity and specificity, was 93.24%, indicating the model's strong overall performance across both classes.

McNemar's test p-value was 1.0, signifying no statistically significant differences between the false positives and false negatives, which further supports the reliability of the predictions.

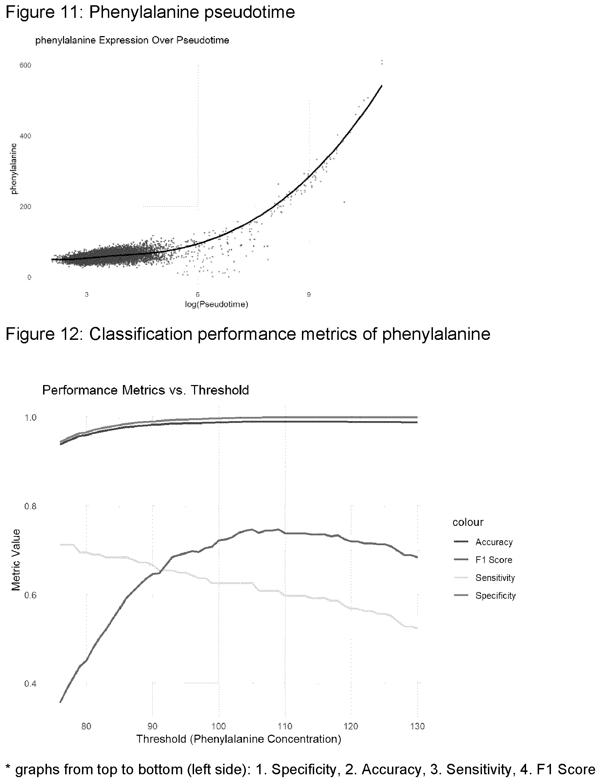

Figure 11 shows the relationship between corrected phenylalanine concentration and log(pseudotime). The black line represents the polynomial fit or trend line, which is increasing as pseudotime progresses.

Summary:

Non-linear progression: The polynomial trend line indicates a non-linear relationship between phenylalanine levels and pseudotime. The upward curve suggests that as pseudotime increases, phenylalanine levels also rise, particularly more steeply after a certain point.

Two distinct regions:

In the early stages of pseudotime (lower values, towards 3), phenylalanine levels remain relatively stable and low.

As pseudotime progresses beyond a certain threshold (around 5-6), the expression of phenylalanine increases dramatically, indicating a strong association at later stages.

Figure 12:

This plot shows the performance of different classification metrics (accuracy, F1 score, sensitivity, and specificity) across a range of phenylalanine concentration thresholds for differentiating between diabetic and non-diabetic states.

Interpretation:

Accuracy (Blue Line):
The accuracy remains high (close to 1.0) across most thresholds, suggesting that the classifier is generally effective at correctly identifying both diabetic and non-diabetic cases.
It starts to level off around 90-100 units of phenylalanine concentration.
F1 Score (Purple Line):
The F1 score initially increases, peaking around a threshold of 90-95, and then begins to decrease as the threshold increases.
This indicates that this range of thresholds (around 90-95) may offer a balance between precision and recall, optimizing the classifier's overall performance.

Sensitivity (Green Line):

Sensitivity declines steadily as the threshold increases. This suggests that higher thresholds reduce the classifier's ability to identify true positive diabetic cases. Higher sensitivity at lower thresholds implies that more diabetic cases are correctly classified, though this may come with an increase in false positives.

Specificity (Red Line):

Specificity remains high and relatively stable across all thresholds, meaning that non-diabetic cases are consistently identified correctly.
This high specificity implies that the classifier effectively minimizes false positives, particularly at higher thresholds.

Summary:

The optimal threshold for differentiating diabetic and non-diabetic states appears to be 105 nmol/mL phenylalanine concentration. This range provides a high F1 score while balancing sensitivity and specificity. Setting the threshold around 105 (or a range of 100-110 nmol/mL) appears to be optimal. This threshold maximizes the F1 score and maintains high accuracy and specificity, making it suitable for reliable diabetes classification with minimized false positives.

Figure 13:

Findings:

Density Distributions: Non-diabetics (green line) and diabetics (red line) show distinct distributions, but there is a significant overlap region (shaded blue), indicating classification challenges.
Threshold Selection: A threshold at log(phenylalanine) ≈ 4.4 (purple line) is identified to balance sensitivity and specificity. This corresponds to a phenylalanine value of 80.8nmol/mL.
This can be interpreted as the threshold og phenylalanine where chances of progressing into diabetes start to increase significantly. Or the value where risk of becoming diabetic begins to become quite significant.

Figure 14:

Findings:

Starting Point of Diagnostic Relevance:
The phenylalanine level of 81 nmol/ml represents the point where the posterior probability of being diabetic begins to rise from nearly 0. Below this level, individuals are highly likely to be non-diabetic, as their posterior probability remains very low.
Onset of the Transition Zone:
This line marks the beginning of the transition zone-the range in which phenylalanine levels start contributing meaningful information about diabetes risk.
Between 81 and 101 nmol/ml (the main cutoff), there is a rapid increase in the probability of being diabetic. In this region, individuals' diabetes risk is progressively higher, though not yet definitive.

Clinical implications

Phenylalanine Levels < 81 nmol/ml: These levels correspond to a very low probability of diabetes. Individuals with phenylalanine levels below 81 are likely to be non-diabetic with high certainty.
Phenylalanine Levels 81-101 nmol/ml: This range is the probability transition zone, where diabetes risk begins to rise. Individuals in this range may not be definitively classified as diabetic but are at increased risk. Clinicians might interpret this as an indication for closer monitoring or additional tests.
Phenylalanine Levels ≥ 101 nmol/ml: At and beyond the main cutoff, the probability of diabetes exceeds 50%, supporting a stronger likelihood of diabetes. Phenylalanine starts to provide diagnostic value at 81 nmol/ml.

Figure 15:

Interpretation of the first derivative

Initial Increase:

At the beginning of the pseudotime range (log(pseudotime) around 5 to 6), the first derivative is negative, indicating a decrease in phenylalanine levels.
As log(pseudotime) increases from 5 to around 6, the derivative shifts towards zero, suggesting that the rate of decrease slows down.

Peak and Stabilization:

Around log(pseudotime) ≈ 6.5, the derivative reaches close to zero, indicating a point of inflection where phenylalanine levels stop decreasing and start to stabilize. This corresponds to phenylalanine levels of 74.2 nmom/ml.
After this point, the derivative remains close to zero, suggesting that phenylalanine levels are relatively stable for the remainder of the pseudotime range (log(pseudotime) > 6).

Biological implication:

This pattern could indicate an early shift in phenylalanine levels that stabilizes as pseudotime progresses. In a biological context, this could represent a metabolic transition or steady state phase, such as an adaptation period during disease progression or treatment.
The shift from negative to near-zero derivative around log(pseudotime) ≈ 6 may represent a significant biological event, such as the end of an initial response phase. The stable, near-zero derivative at later pseudotimes suggests a period where phenylalanine levels are not changing significantly, potentially indicating a stable metabolic state.

Figure 16:

Interpretation of the second derivative graph

This plot illustrates the second derivative of phenylalanine levels with respect to log-transformed pseudotime, capturing the acceleration or deceleration in phenylalanine levels as pseudotime progresses. Key changes in the second derivative can indicate metabolic shifts or critical points in biological processes.
Early Phase (Before log(pseudotime) = 7.9): This phase shows a slowing rate of increase in phenylalanine levels, possibly corresponding to an initial buildup phase in metabolic activity.
Transition Point (log(pseudotime) = 7.9): This minimum represents an inflection point where phenylalanine levels shift from a decelerating trend to an accelerating one. This point might mark a critical biological transition in the metabolic or disease progression pathway.
Late Phase (After log(pseudotime) = 7.9): The increase in the second derivative suggests renewed acceleration in phenylalanine levels, potentially marking a second phase of metabolic activity or progression.

Figure 17:

This plot shows phenylalanine levels as they change over log-transformed pseudotime, with an inflection point marked on the curve. The inflection point represents a key transition in the trend of phenylalanine expression and corresponds to a phenylalanine concentration of approximately **162.28 nmol/ml**.

Clinical or Biological Implications:

Threshold Value: The phenylalanine level of 162.28 nmol/ml could serve as a threshold indicating a shift in metabolic state. Values near or above this level might correspond to a significant stage in disease progression or a change in metabolic activity.

This inflection point highlights a potential **transition period** where close monitoring or intervention might be warranted, as it marks a shift toward accelerated phenylalanine expression and possibly advanced diabetes type 2.

Figure 18:

ROC curve with AUC value, using the optimal value of the pseudotime.

Other literature:

Trapnell, C., Cacchiarelli, D., Grimsby, J. et al. The dynamics and regulators of cell fate decisions are revealed by pseudotemporal ordering of single cells. Nat Biotechnol 32, 381-386 (2014). https://doi.org/10.1038/nbt.2859

Qiu, X., Mao, Q., Tang, Y. et al. Reversed graph embedding resolves complex single-cell trajectories. Nat Methods 14, 979-982 (2017). https://doi.org/10.1038/nmeth.4402

**Claims**

1. A method for predicting or monitoring or stratifying diabetes mellitus comprising

   (a) providing, for each of a plurality of subjects a sample and determining the amount of at least one amino acid in each sample
   (b) providing an amino acid pattern of at least one amino acid from (a) and providing a data set configured to combine the said amino acid pattern with at least one feature of the subject selected from the group consisting of sex, age, height, weight, body mass index
   (c) performing a preprocessing on the data set of (b), preferably using at least propensity score weighting
   (d) calculating one or more mathematical distances, in particular Mahalanobis distances from the data set of (c); and
   (e) i.) identifying a subject at risk or ii.) differentiating between a non-diabetic and a diabetic subject.

2. A method for predicting or monitoring or stratifying diabetes mellitus comprising

   (a) providing, for each of a plurality of subjects a sample and determining the amount of at least one amino acid in each sample
   (b) providing an amino acid pattern of at least one amino acid from (a) and providing a data set configured to combine the said amino acid pattern with at least one feature of the subject selected from the group consisting of sex, age, height, weight, body mass index
   (c) performing a preprocessing on the data set of (b), preferably using at least propensity score weighting
   (d) calculating one or more mathematical distances, in particular Mahalanobis distances from the data set of (c); and
   (e) using a classification system, wherein the classification system is a machine-learning classifier, and wherein the data set is configured from (b) and/or (d) and classifying a subject at risk.

3. A method for predicting or monitoring or stratifying diabetes mellitus, wherein step (d) comprises the step, and calculating one or more pseudotime values therefrom.

4. A method for predicting or monitoring or stratifying diabetes mellitus, wherein the risk reflects the metabolic progression of diabetes.

5. A method for predicting or stratifying diabetes mellitus according to claim 2, wherein the machine-learning classifier is selected from the group consisting of Extreme Gradient Boosting (XGBoost), Naive Bayes (NB), K-Nearest Neighbors (KNN), Random Forest (RF), and Decision Trees (DTs), k-Nearest Neighbors (k-NN), Centroid-Based Classifiers, Minimum Distance Classifier and Support Vector Machines (SVMs), if required, with Distance-Based Kernels.

6. A method for predicting or stratifying diabetes mellitus according to any of the preceding claims, wherein the amino acid is at least one selected from the group consisting of phenylalanine, tyrosine, tryptophan, histidine, alanine, ornithine, arginine.

7. A method for the diagnosis, prognosis or prediction of diabetes mellitus comprising

providing a biological sample of a subject and determining the amount of phenylalanine,
wherein a threshold value of less than 77 - 83 nmol/ml, in particular less than 81 nmol/ml is indicative for a non-diabetic status,
and/or
wherein a threshold value of 81 - 105 nmol/ml is indicative for a pre-diabetic status,
and/or
wherein a threshold value of more than 105 - 162 nmol/ml is indicative for a moderate diabetic status,
and/or
wherein a threshold value of more than 162 nmol/ml is indicative for an advanced diabetic status,
wherein all mentioned values may deviate by +/- 10 nmol/m.

8. A method for the diagnosis, prognosis or prediction of diabetes mellitus according to claim 7, **characterized in that** the diagnosis takes place for prognosis, for prophylaxis, for early detection and detection by means of differential diagnosis, for assessment of the degree of severity, and for assessing the course of diabetes mellitus, particularly Type II diabetes mellitus, and its concomitant illnesses and sequelae, as an accompaniment to therapy, and for assessing clinical decisions, particularly further treatment by means of medications for the treatment or therapy of diabetes mellitus, particularly Type II diabetes mellitus, and its concomitant illnesses and sequelae.

9. A method for the diagnosis, prognosis or prediction of diabetes mellitus according to claim 7, wherein the threshold values are determined by a method according to claims 1 to 6.

Figure 1: Distributional balance of propensity scores for phenylalanine

Figure 2: Covariate Balancing for the prediction of phenylalanine

Figure 3: Distribution of pseudotime values in the diabetic vs non-diabetic observations

Figure 4: Visualization of performance metrics for pseudotime-based classification

Figure 5: Density of pseudotime distributions in reference to $log(pseudotime) = 5.54$

Density Curves of Diabetic vs Non-diabetic with Pseudotime I

Figure 6: Confusion matrix for log(pseudotime)=5.54

```
Confusion Matrix and Statistics

                    Actual
Predicted       Non-diabetic Diabetic
  Non-diabetic           7053       20
  Diabetic                 53      154

              Accuracy : 0.99
                95% CI : (0.9874, 0.9921)
   No Information Rate : 0.9761
   P-Value [Acc > NIR] : < 2.2e-16

                 Kappa : 0.8033

Mcnemar's Test P-Value : 0.0001802

           Sensitivity : 0.88506
           Specificity : 0.99254
        Pos Pred Value : 0.74396
        Neg Pred Value : 0.99717
            Prevalence : 0.02390
        Detection Rate : 0.02115
  Detection Prevalence : 0.02843
     Balanced Accuracy : 0.93880

      'Positive' Class : Diabetic
```

Figure 7: Validating log(pseudotime)=5.54 on validation data

```
Confusion Matrix and statistics

                    Reference
Prediction      Non-diabetic Diabetic
  Non-diabetic            549        2
  Diabetic                  1       13

               Accuracy : 0.9947
                 95% CI : (0.9846, 0.9989)
    No Information Rate : 0.9735
    P-Value [Acc > NIR] : 0.0001852

                  Kappa : 0.8938

 Mcnemar's Test P-Value : 1.0000000

            Sensitivity : 0.86667
            Specificity : 0.99818
         Pos Pred Value : 0.92857
         Neg Pred Value : 0.99637
             Prevalence : 0.02655
         Detection Rate : 0.02301
   Detection Prevalence : 0.02478
      Balanced Accuracy : 0.93242

       'Positive' Class : Diabetic
```

## Figure 8: Best predictors of the log-pseudotime

Best predictors of the Pseudotime

Figure 9. Predictors of diabetes

Figure 10. Validation of diabetes prediction model on validation data

```
                   Confusion Matrix and Statistics

                          Non-diabetic Diabetic
          Non-diabetic             549        2
          Diabetic                   1       13

                      Accuracy : 0.9947
                        95% CI : (0.9846, 0.9989)
           No Information Rate : 0.9735
           P-Value [Acc > NIR] : 0.0001852

                         Kappa : 0.8938

        Mcnemar's Test P-Value : 1.0000000

                   Sensitivity : 0.86667
                   Specificity : 0.99818
                Pos Pred Value : 0.92857
                Neg Pred Value : 0.99637
                    Prevalence : 0.02655
                Detection Rate : 0.02301
          Detection Prevalence : 0.02478
             Balanced Accuracy : 0.93242

              'Positive' Class : Diabetic
```

Figure 11: Phenylalanine pseudotime

phenylalanine Expression Over Pseudotime

Figure 12: Classification performance metrics of phenylalanine

* graphs from top to bottom (left side): 1. Specificity, 2. Accuracy, 3. Sensitivity, 4. F1 Score

Figure 13: Density Analysis of Log-Transformed Phenylalanine Levels for Diabetes Classification

**Density Comparison of Log-Transformed Phenylalanine Levels**

Legend:
- Mixture Density
- Diabetics
- Non-Diabetics
- Overlap
- Threshold

Threshold

Y-axis: Density of Log (Phenylalanine)
X-axis: Log-Transformed Phenylalanine

Figure 14: Danger zone of diagnostic relevance based on phenylalanine

**Posterior Probability of Being Diabetic vs. Phenylalanine Levels**

Cutoff = 101

Y-axis: Posterior Probability of Being Diabetic
X-axis: Phenylalanine Level

Figure 15: First derivative of phenylalanine levels with respect to pseudotime

**First Derivative of Phenylalanine wrt Pseudotime**

Figure 16: Second derivative of phenylalanine levels with respect to pseudotime

**Second Derivative of Phenylalanine with respect to log(Pseudotime)**

Figure 17: Second derivative of phenylalanine levels with respect to pseudotime

**Phenylalanine Expression with Inflection Point**

Figure 18: ROC curve with AUC value, using the optimal value of the pseudotime.

ROC Curve Using Optimal Pseudotime Threshold Value of 5.5

AUC = 0.997

Europäisches
Patentamt

European
Patent Office

Office européen
des brevets

# EUROPEAN SEARCH REPORT

**Application Number**

EP 25 15 2904

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SU XIULI ET AL: "Ten metabolites-based algorithm predicts the future development of type 2 diabetes in Chinese", JOURNAL OF ADVANCED RESEARCH, ELSEVIER, AMSTERDAM, NL, vol. 64, 28 November 2023 (2023-11-28), pages 131-142, XP087613379, ISSN: 2090-1232, DOI: 10.1016/J.JARE.2023.11.026 [retrieved on 2023-11-28] | 1-6 | INV. G16B20/00 G16B40/20 G16H50/20 G16H50/30 |
| A | * the whole document * | 7-9 | |
| X | THOMAS J WANG ET AL: "Metabolite profiles and the risk of developing diabetes", NATURE MEDICINE(AUTHOR MANUSCRIPT ), vol. 17, no. 4, 20 March 2011 (2011-03-20) , pages 448-453, XP055417419, New York ISSN: 1078-8956, DOI: 10.1038/nm.2307 | 1-6 | |
| A | * the whole document * | 7-9 | |
| A | MERINO JORDI ET AL: "Metabolomics insights into early type 2 diabetes pathogenesis and detection in individuals with normal fasting glucose", DIABETOLOGIA, SPRINGER BERLIN HEIDELBERG, BERLIN/HEIDELBERG, vol. 61, no. 6, 6 April 2018 (2018-04-06), pages 1315-1324, XP036751527, ISSN: 0012-186X, DOI: 10.1007/S00125-018-4599-X [retrieved on 2018-04-06] * the whole document * | 1-9 | TECHNICAL FIELDS SEARCHED (IPC)<br><br>G16B<br>G01N<br>G16H |

-/--

The present search report has been drawn up for all claims

| Place of search | Date of completion of the search | Examiner |
|---|---|---|
| Munich | 22 September 2025 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................

& : member of the same patent family, corresponding
document

EPO FORM 1503 03.82 (P04C01)

Europäisches
Patentamt

European
Patent Office

Office européen
des brevets

## EUROPEAN SEARCH REPORT

**Application Number**

EP 25 15 2904

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SIOMKAJLO MARTA ET AL: "Specific plasma amino acid disturbances associated with metabolic syndrome", ENDOCRINE, HUMANA PRESS, INC, US, vol. 58, no. 3, 26 October 2017 (2017-10-26), pages 553-562, XP036366463, ISSN: 1355-008X, DOI: 10.1007/S12020-017-1460-9 [retrieved on 2017-10-26] * the whole document * | 1-9 | |
| A | WU HAO ET AL: "The association between circulating phenylalanine and the temporal risk of impaired insulin markers in gestational diabetes mellitus", MOLECULAR GENETICS AND METABOLISM REPORTS, vol. 40, 1 September 2024 (2024-09-01), page 101090, XP093317630, ISSN: 2214-4269, DOI: 10.1016/j.ymgmr.2024.101090 Retrieved from the Internet: URL:https://pmc.ncbi.nlm.nih.gov/articles/PMC11227027/pdf/main.pdf> * the whole document * | 1-9 | TECHNICAL FIELDS SEARCHED (IPC) |

-/--

The present search report has been drawn up for all claims

| Place of search | Date of completion of the search | Examiner |
|---|---|---|
| Munich | 22 September 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHOU QIAN ET AL: "Phenylalanine impairs insulin signaling and inhibits glucose uptake through modification of IR[beta]", NATURE COMMUNICATIONS, vol. 13, no. 1, 25 July 2022 (2022-07-25), XP093317623, UK ISSN: 2041-1723, DOI: 10.1038/s41467-022-32000-0 Retrieved from the Internet: URL:https://pmc.ncbi.nlm.nih.gov/articles/ PMC9314339/pdf/41467_2022_Article_32000.pd f> * the whole document * | 7-9 | |

-----

TECHNICAL FIELDS
SEARCHED (IPC)

The present search report has been drawn up for all claims

| Place of search | Date of completion of the search | Examiner |
|---|---|---|
| Munich | 22 September 2025 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................................
& : member of the same patent family, corresponding
document

EPO FORM 1503 03.82 (P04C01)

Europäisches
Patentamt

European
Patent Office

Office européen
des brevets

**Application Number**

EP 25 15 2904

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 25 15 2904

The Search Division considers that the present European patent application does not comply with the requirements of unity of invention and relates to several inventions or groups of inventions, namely:

    **1. claims: 1-6**

        Method of calculating the pseudotime metric for a group of individuals.
            ---

    **2. claims: 7-9**

        Method of improving diabetes classification accuracy of a patient.
            ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106979982 A

**Non-patent literature cited in the description**

- Global report on diabetes. World Health Organization, 2016
- **KAHN SE ; COOPER ME ; DEL PRATO S**. Pathophysiology and treatment of type 2 diabetes: perspectives on the past, present, and future.. *Lancet*, 2014
- **DASHDONDOV, K.** ; **LEE, S.** ; **ERDENEBAT, M.-U.** Enhancing Diabetes Prediction and Prevention through Mahalanobis Distance and Machine Learning Integration. *Appl. Sci.*, 2024, vol. 14, 7480
- **MAHALANOBIS, P.C.** On the Generalised Distance in Statistic. *Sankhya A*, 1936, vol. 80 (1), 1-7, https://doi.org/10.1007/s13171-019-00164-5
- **KUHN M** ; **WICKHAM H** ; **HVITFELDT E**. *recipes: Preprocessing and Feature Engineering Steps for Modeling*, 2024, https://recipes.tidymodels.org
- **CHESNAYE NC** ; **STEL VS** ; **TRIPEPI G** ; **DEKKER FW** ; **FU EL** ; **ZOCCALI C** ; **JAGER KJ**. An introduction to inverse probability of treatment weighting in observational research. *Clin Kidney J*, 26 August 2021, vol. 15 (1), 14-20
- **TRAPNELL, C.** ; **CACCHIARELLI, D.** ; **GRIMSBY, J. et al.** The dynamics and regulators of cell fate decisions are revealed by pseudotemporal ordering of single cells. *Nat Biotechnol*, 2014, vol. 32, 381-386, https://doi.org/10.1038/nbt.2859
- **QIU, X.** ; **MAO, Q.** ; **TANG, Y. et al.** Reversed graph embedding resolves complex single-cell trajectories. *Nat Methods*, 2017, vol. 14, 979-982, https://doi.org/10.1038/nmeth.4402